# EUROPEAN PATENT SPECIFICATION

(11) **EP 2 809 793 B1**
(45) Date of publication and mention of the grant of the patent: **30.10.2019**
(21) Application number: 13725757.2
(22) Date of filing: 24.01.2013
(51) Int. Cl.: C12P 19/12, C12P 19/14, C12P 19/16, C12P 19/22, C07H 15/04

(54) **PROCESS FOR PRODUCING MALTITOL FROM STARCH**
VERFAHREN ZUR HERSTELLUNG VON MALTIT AUS STÄRKE
PROCÉDÉ DE PRODUCTION DE MALTITOL À PARTIR D'AMIDON

(30) Priority: 31.01.2012 EP 12000624; 29.02.2012 EP 12001377
(43) Date of publication of application: 10.12.2014
(73) Proprietor: Cargill, Incorporated, Wayzata, MN 55391 (US)
(72) Inventor: FURLAN, Tiziano, I-45100 Rovigo (IT); NATALONI, Luigi, I-40139 Bologna (IT); TOLOMELLI, Patrizia, I-44124 Ferrara (IT)
(74) Representative: Jantschy, Jasmin
(86) International application number: PCT/IB2013/000962
(87) International publication number: WO 2013/114223

(56) References cited:
- EP-A1- 0 816 373
- WO-A2-2005/014608
- DE-A1- 1 935 330
- US-A- 4 487 198
- DATABASE WPI Week 200257 Thomson Scientific, London, GB; AN 2002-531597 XP002681976, -& JP 2002 101896 A (NIPPON SHIRYO KOGYO KK) 9 April 2002 (2002-04-09)

## Description

### Field of the Invention

The present invention relates to a process for preparing high purity liquid maltitol products.

### Background of the Invention

Methods allowing the production of maltitol rich syrups are already well-known.

US 5,873,943 provides an economical advantageous process for manufacturing crystalline maltitol. The process uses a product having a maltose purity of 81 to 90% as the starting material. The syrup is hydrogenated and then subjected to a chromatographic separation resulting in an aqueous solution of maltitol having a maltitol purity of 94 to 99.9%. The aqueous solution is further crystallized in the presence of a seed crystal.

EP 1 656 388 relates to a process for preparing maltitol enriched products and the process is chromatographically fractionating a maltose syrup followed by hydrogenating it into a liquid maltitol enriched product and optionally solidifying or crystallizing the maltitol. Liquid, solid and crystalline maltitol of different purities are obtainable by a single process.

WO 2008/029033 relates to a method for obtaining a syrup with a high maltitol content and the invention is more particularly applicable in the field of the agrofoods industry.

DE 19 35 330 A1 discloses a process for preparing a maltitol containing syrup from starch. Here, the alpha-amylase used in the liquefaction is inactivated before the saccharification and then the saccharification is carried out in the presence of beta-amylase and the debranching enzyme pullulanase for obtaining a maltose containing syrup comprising at least 85% maltose based on dry matter and less than 1.5% glucose based on dry matter.

JP 2002 101896 A discloses a method for the production of a maltose liquid.

There is still a further need of having a process providing a syrup rich in maltitol and low in sorbitol and low in maltotriitol.

### Summary of the Invention

The current invention relates to a process for preparing a maltitol containing syrup comprising the successive steps of
a) Carrying out liquefaction of a starch milk,
b) Carrying out saccharification of the liquefied starch milk in the presence of alpha-amylase, and beta-amylase and a debranching enzyme selected from the group of pullulanase, iso-amylase and mixtures thereof,
c) Further adding maltogenic alpha-amylase and/or iso-amylase, optionally followed by demineralisation of maltose containing syrup,
d) Molecular sieving of the maltose containing syrup to obtain a fraction (A) comprising at least 95% maltose based on dry substance of fraction (A),
e) Hydrogenating catalytically fraction (A) for obtaining a liquid maltitol enriched product (B),
wherein in step b) the saccharification is taking place in presence of from 1% to 4% of residual activity of total amount of alpha-amylase applied in the liquefaction of step a);
and wherein after step c) additional alpha-amylase is added at about 70 to 85% spent time of total saccharification time, for obtaining a maltose containing syrup comprising at least 85% maltose based on dry matter and less than 1.5% glucose based on dry matter.

### Detailed Description

The current invention relates to a process for preparing a maltitol containing syrup comprising the successive steps of
a) Carrying out liquefaction of a starch milk,
b) Carrying out saccharification of the liquefied starch milk in the presence of alpha-amylase, and beta-amylase and a debranching enzyme selected from the group of pullulanase, iso-amylase and mixtures thereof, preferably pullulanase,
c) Further adding maltogenic alpha-amylase and/or iso-amylase, optionally followed by demineralisation of maltose containing syrup
d) Molecular sieving of the maltose containing syrup to obtain a fraction (A) comprising at least 95% maltose based on dry substance of fraction (A),
e) Hydrogenating catalytically fraction (A) for obtaining a liquid maltitol enriched product (B),
wherein in step b) the saccharification is taking place in presence of from 1% to 4% of residual activity of total amount of alpha-amylase applied in the liquefaction;
and wherein after step c) additional alpha-amylase is added at about 70 to 85% spent time of total saccharification time, for obtaining a maltose containing syrup comprising at least 85% maltose based on dry matter and less than 1.5% glucose based on dry matter.

The liquefaction is carried out in presence of alpha-amylase.

The liquefaction and saccharification of starch can be conducted in various ways, but the current invention demonstrates that combining the liquefaction with a specific saccharification step allows obtaining a maltose syrup comprising at least 85% maltose (= DP2), or at least 87%, at least 90% maltose based on dry matter and less than 1.5% glucose (= DP1) based on dry matter, preferably less than 1% glucose based on dry matter, and preferably comprising less than 10% DP3, more preferably comprising less than 10% of oligosaccharides having a polymerisation degree of 3 or more (= DP3+).

The liquefaction is conducted on starch of any botanical origin. For instance it may originate from wheat, corn or potato.

The liquefaction is to be considered as a controlled hydrolysis of starch milk, in the presence of enzymes such as alpha-amylase, and so as to obtain a liquefied starch milk with a low degree of conversion. Thus the conditions of temperature, pH, enzyme (type as well as concentration) are selected in such a way that they make it possible to obtain a DE (= dextrose equivalent) of not more than 6, preferably from 4 to 5.

Preferably the liquefaction is carried out in three steps, the first step is consisting in heating the starch milk at a temperature in the range of 105 to 108°C and in presence of a thermostable alpha-amylase for a few minutes, typically from 8 to 15 minutes, not longer than 20 minutes. The second step is consisting of heating the starch milk thus treated at a temperature in the range of 140 to 160°C, preferably in the range of 145-155°C for a few minutes, for a time period of 5 to 8 minutes, but no longer than 20 minutes. After cooling down to about 95 to 100°C, a second small dosage of alpha-amylase is added and the liquefaction continued for another 30 to 50 minutes and thus is tuned so to achieve a starch slurry with a D.E. of 4 to 6, preferably from 4 to 5.

The liquefaction according to the current invention allows preparing a D.E. of 4 to 6, preferably from 4 to 5, wherein the composition of the oligosaccharides (DPn) is pre-fine-tuned for the subsequent saccharification.

Once the liquefaction step is ended, a controlled inhibition is conducted such that only a partial inhibition of the alpha-amylase is carried out and residual alpha-amylase is maintained for the subsequent saccharification step. Preferably the partial inhibition is conducted at a pH of 3.5 to 4 at a temperature not higher than 100°C. Preferably the partial inhibition is taking place during a time period of 1 to 10 minutes. The residual (remaining active) alpha-amylase is further used in the subsequent saccharification step. Preferably, the residual alpha-amylase corresponds to from 5 to 15% of the total amount added in the second dosing of the liquefaction. Finally, the residual alpha-amylase corresponds to 7% to 12% of the total amount added in the second dosing of the liquefaction.

Compared with the actual total amount of alpha-amylase added during the liquefaction (= dose 1 + second dosage) it corresponds to 1% to 4%, preferably from 1.4% to 3% of residual activity of total amount of alpha-amylase.

Preferably the saccharification of liquefied starch milk is carried out in presence of alpha-amylase, and beta-amylase and as debranching enzyme, pullulanase, wherein the saccharification is taking place in presence of residual amount of alpha-amylase applied in the liquefaction of step a), in presence of from 1% to 4%, or in presence of 1.4% to 3% of residual activity of total amount of alpha-amylase applied in the liquefaction.

Saccharification is then continued by adding a beta-amylase and a debranching enzyme selected from the group of pullulanase, iso-amylase and mixtures thereof. Preferably pullulanase is added. The addition of debranching enzyme makes it possible to hydrolyse the 1,6- linkages and thus to reduce the quantity of highly branched oligosaccharides. Preferably the ratio of beta-amylase to debranching enzyme is from 1:1 to 1:4. Preferably the ratio of beta-amylase to pullulanase is from 1:1 to 1:4. Ratios from 1:1 to 1:5 or even up to 1:10 are part of the invention. Preferably, in applying pullulanase as debranching enzyme, the ratio of beta-amylase to pullulanase is from 1:2 to 1:4 and preferably the higher upper-end from 1:3 to 1:4 is applied.

Maltogenic alpha-amylase and/or iso-amylase is added to the so far treated starch milk, at about 20 to 50% spent time of the total saccharification time, preferably at about 25 to 35%, preferably at about 25% to 30% spent time of the total saccharification time. The maltogenic alpha-amylase is an exo-acting alpha-amylase which is responsible for the exo-hydrolysis of 1,4-alpha-glucosidic linkages. Iso-amylase is a debranching enzyme which is hydrolysing the 1,6-linkages and reduces the amount of the reversion products.

In a typical process the total saccharification time is about 16 to 30 hours, preferably from 20 to 24 hours, and the maltogenic alpha-amylase and/or iso-amylase is added after 7 to 8 hours of saccharification time.

The saccharification is thus continued until a maltose rich syrup is obtained which is containing at least 85% maltose based on dry matter and less than 1.5% glucose based on dry matter, preferably less than 1% glucose based on dry matter.

The saccharification is conducted such that a syrup rich in maltose is obtained such that it contains at least 85% maltose on dry matter, or at least 87% maltose, at least 90% maltose based on dry matter and less than 1.5% glucose based on dry matter, preferably less than 1% glucose based on dry matter and less than 10% of DP3 or less than 10% of polymers having a degree of polymerisation of 3 or more (= DP3+) based on dry matter, preferably less than 5% DP3+. Even more preferably the polymers having a degree of polymerisation higher than 3 are negligible and the amount of polymers having a degree of polymerisation of 3 is below 5%, more preferably below 3%, most preferably below 1% based on dry matter of the syrup.

Finally more towards the end of the saccharification step, additional alpha-amylase is added. This specific low amount may further improve the subsequent down-streaming process. The alpha-amylase is added at about 70 to 85% spent time of total saccharification time, preferably at about 80 to 83% spent time of the total saccharification time. This can correspond to about 4 hours before ending the saccharification process.

The process of the current invention allows to obtain product with very high content (= at least 85%, 87%, 90%) of maltose while the content of glucose is below 1.5%, with low DP3 amount and wherein the presence of long chain oligosaccharides is reduced. The composition of DPn is different from the composition that is usually obtained after liquefaction and saccharification. In particular the use of residual alpha-amylase in the subsequent saccharification step and the further addition of alpha-amylase towards the end of the saccharification contributes to the change of the composition of the DPn (oligosaccharide) fraction. The amount of the higher oligosaccharides (= longer chains) is reduced.

The thus obtained saccharified syrup can be purified according to the well-known demineralization processes such as by applying ion exchange resins. Alternatively, the saccharified syrup may be filtered on a precoat filter or by microfiltration on membranes and then followed by demineralization.

So far high maltose (up to 80%) syrups with low amount of glucose have been obtained, as well as very high maltose (up to 90%) with significant amounts of residual glucose (5 to 7%). The current invention has demonstrated that by applying the liquefaction according to the current process and combining it with the saccharification step as is claimed in the current invention, surprisingly, it is feasible to obtain maltose syrups with very high content of maltose (at least 85%, at least 87%, at least 90%) and low amounts of glucose (less than 1.5%, less than 1%). And finally also the content of DP3 is low, less than 10%, preferably less than 5%. Furthermore the DPn fraction starting with DP4 has a significant different composition so that the amount of long chain oligosaccharides is reduced. This changed composition makes the final product of the current invention more stable and it is a better precursor for maltitol production through hydrogenation. Either the time of the hydrogenation step can be significantly reduced or less catalyst is required under the same hydrogenation conditions.

The maltose containing syrup obtained after saccharification is subjected to a molecular sieving step. This molecular sieving can be a stage of separation on membranes or a chromatographic fractionation. In the process according to the invention it is possible to use in the stage of separation on membranes a stage of nanofiltration on membranes. Membranes with different diameters of pore are commercially available and are described in numerous patent applications.

The chromatographic fractionation is carried out either discontinuously or continuously (simulated moving bed), on adsorbents such as ionic resins, or zeolites, preferably cation resins are applied. Preferably the cationic resins are charged with alkali or earth alkali ions, more preferably with aid of sodium ions.

By applying the same or similar conditions in the chromatographic fractionation, in respect of column design, resin type, temperature of feed material, flow-rate, dry matter of feed material and the like, as used for the chromatographic fractionation of product in EP 1 656 388, the yield of the fraction enriched in maltose is increased with at least 5%, preferably at least 10%. The yield is calculated as the amount of fraction enriched in maltose times dry matter of fraction, and divided by the amount of feed times dry matter of feed, and everything multiplied with 100 in order to express in percentage.

This means that by obtaining a maltose containing syrup with very high content of maltose (at least 85%, 87%, 90%) and low amounts of glucose (less than 1.5%, less than 1%), and finally also with the content of DP3 less than 10%, preferably less than 5%, the yield of the subsequent chromatographic fractionation is increased with at least 5%, preferably at least 10%.

Further disclosed is the use of a maltose containing syrup comprising at least 85% maltose, or at least 87%, at least 90% maltose based on dry matter and less than 1.5% glucose based on dry matter and less than 10% DP3 based on dry matter, for example less than 1% glucose based on dry matter, for increasing the yield of a chromatographic fractionation with at least 5%, for example at least 10%. Disclosed is a method to increase the yield of a chromatographic fractionation of maltose containing syrups by applying a maltose containing syrup comprising at least 85% maltose, or at least 87%, at least 90% maltose based on dry matter and less than 1.5% glucose based on dry matter and less than 10% DP3 based on dry matter, for example less than 1% glucose based on dry matter.

The thus obtained fraction (A) (= fraction enriched in maltose) comprising at least 95%, preferably at least 96%, preferably at least 97%, more preferably at least 98% maltose based on dry substance of fraction (A) is hydrogenated in presence of hydrogenation catalysts. Preferably a Raney nickel based catalyst is used as hydrogenation catalyst.

Any hydrogenation condition can be suitable in as far there is no decomposition of maltose taking place. Usually the hydrogenation step is conducted at hydrogen gas pressure of at least 10 bar, preferably between 30 to 200 bar and at a temperature of 90 to 150°C so that the hydrogenation continues until the absorption of hydrogen gas stops.

The supply syrup = fraction (A) can be used at dry substance of at least 50%, activated nickel catalyst is added and the hydrogenation is taking place at a temperature up to 135°C and hydrogen pressure of at least 40 bar. By applying fraction (A) comprising at least 95% maltose and obtainable by the process of the current invention, the amount of activated nickel catalyst in the hydrogenation step can be reduced with at least 5%, preferably at least with 10%. Usually (see EP 1 656 388) the activated nickel catalyst is added in an amount of 4% on dry mater of supply syrup. In the current invention, the activated nickel catalyst is added in an amount of 3.6% on dry matter of supply syrup (A). Preferably, the change of the composition of the DPn (oligosaccharides) fraction has a beneficial effect on the hydrogenation.

The current invention relates to the use of a maltose containing syrup comprising at least 85% maltose or at least 87%, at least 90% maltose based on dry matter and less than 1.5% glucose based on dry matter and less than 10% DP3 based on dry matter, preferably less than 1% glucose based on dry matter for decreasing the amount of catalyst, preferably activated nickel in hydrogenation step with at least 5%, preferably at least 10%.

It relates to a method to decrease the amount of catalyst, preferably activated nickel catalyst in hydrogenation of maltose containing syrups by applying a maltose containing syrup comprising at least 85% maltose or at least 87%, at least 90% maltose based on dry matter and less than 1.5% glucose based on dry matter and less than 10% DP3 based on dry matter, preferably less than 1% glucose based on dry matter.

After the completion of absorption of hydrogen gas, e.g. after about 3 hours of hydrogenation, the hydrogenation catalyst (=activated nickel catalyst) is removed from the resulting liquid maltitol product (B). This syrup can be further decolorized and/or de-ionized by activated carbon or ion-exchange resin and/or polisher resins.

The current invention further relates to a maltitol containing syrup containing at least 95% maltitol preferably at least 96%, more preferably at least 97% based on dry matter, and less than 1.2% sorbitol based on dry matter, and having a dry matter content of 50-75%, preferably a dry matter content of 55-70% and said maltitol containing syrup being obtained by the process of the current invention. Preferably said maltitol containing syrup is containing less than 1.1%, less than 1.0% sorbitol. It further relates to a maltitol containing syrup which is further containing less than 10% hydrogenated DP3 based on dry matter.

This syrup can be used in food applications or industrial applications, or as precursor for solidified or crystalline maltitol or a feed material in chromatographic purification.

The invention will hereunder be illustrated in the form of the following examples.

### Examples

### Example 1

### Liquefaction

Starch slurry at dry matter content between 27-35% ds (is dry matter) was liquefied, after pH adjustment at 5,8(±1) and after dosage of 0,08-0,1% of alpha-amylase (Spezyme (Genencor)) by using jet cooker at 108°C. After 8-15 minutes, the pasting temperature was reduced to 100°C by atmospheric flash and then the slurry was sent to the second jet at 152°C. After 5-8 minutes of pasting, the slurry was cooled down to 100°C and a second dosage (0,025%) of the same alpha-amylase was added and this amount is tuned in order to reach 4-6 DE (target 4,5).

After 30-50 minutes of reaction on the agitated column at 100°C, the pH of the liquefact was adjusted at 3-4 (target 3.5-4) at 100°C for max 10 minutes to inhibit part of the alpha-amylase. After this treatment, 7 to 10% of the alpha-amylase added as second dosage was maintained.

### Example 2

### Saccharification - RECIPE 1

The product of example 1 was used. Saccharification started at pH 4,8-5,0 in presence of residual alpha-amylase and 0,1% of beta-amylase (Optimalt BBA (Genencor)) and 0,4% of pullulanase (Promozyme D2 (Novozyme)). After 7-8h reaction 0,02% of maltogenic alpha-amylase (Maltogenase (Novozyme)) was added.

At least 4 hours before unloading the saccharificator, 0,1-0,2% of alpha-amylase (Liquozyme X (Novozyme)) was added..After a total saccharification time of 24-30h the following composition was reached: glucose <1%, maltose (= DP2) 85-87%, DP3 (= oligosaccharide with polymerization degree of 3) 7-10%, DP4+ (oligosaccharides with polymerization degree of 4 and more) < 5%.

Purification is carried out as the purification for regular glucose syrups.

### Example 3

### Saccharification - RECIPE 2

The product of example 1 was used. Saccharification started at pH 4,8-5,0 in presence of residual alpha-amylase and 0,1 % of beta-amylase (Optimalt BBA (Genencor)) and 0,4% of pullulanase (Promozyme D2 (Novozyme)). and 0,1% of iso-amylase. After 7-8h reaction 0,1% maltogenic alpha-amylase (Maltogenase (Novozyme)) was added.

At least 4 hours before unloading the saccharificator, 0,1-0,2% of alpha-amylase (Liquozyme X (Novozyme)) was added. After a total saccharification time of 24-30h the following composition was reached: glucose <1%, maltose (=DP2) 87-90%, and DP3 is 4 to 6%.

### Example 4

### Chromatographic fractionation

The product (coming from Recipe 1) with composition (DP1: < 1.0% (=0.9%); DP2: 87%(=86.9%); DP3: 7.5% and DP4+<5 (=4.7%))was concentrated to 60% dry matter.

The concentrated product was applied at 75°C onto a chromatographic equipment (ISMB) with ion exchange resin Dianion UBK 550 in Sodium form, for obtaining a fraction enriched in maltose. Said product had the following composition (DP1: < 1.0%; DP2: 96-98%; DP3: <2%; DP4<1).

HPLC-analysis (Bio-Rad Aminex HPX-87, cation exchange column is the calcium form, column temperature: 80°C, Eluent Flow Rate: 0.6 ml/minute, column pressure limit: 1200 psi, injection volume: 20 µL, pressure control limit about 200 psi above the normal operating pressure of the column, eluent: degassed Milli-Q Purified water, detector: Differential refractometer)

**Table 1**

| | | Feed | Product enriched in maltose | Co-product | Water |
|---|---|---|---|---|---|
| Composition (%) | DP1 | 0.9 | 0.8 | 1.4 | |
| | DP2 | 86.9 | 96.6 | 44 | |
| | DP3 | 7.5 | 1.7 | 32 | |
| | DP4+ | 4.7 | 0.9 | 21.6 | |
| Total weight (kg/h) | | 37.6 | 47 | 65 | 74.4 |
| Flow rate (L/h) | | 29.00 | 40.17 | 63.11 | 74.2 |
| % d.s. | | 60.0 | 39 | 6.5 | |

| | | | | | |
|---|---|---|---|---|---|
| The yield of the product enriched in maltose is (total weight * % d.s. product *100/ total weight * % d.s of feed) = 81.2%. | | | | | |

### Comparative example 4 - Chromatographic fractionation - see EP 1 656 388

The product with composition (DP1: 1.5%; DP2: 80.0%; DP3: 12.5% and DP4+: 6%) was concentrated to 60% dry matter which is obtained in EP 1 656 388.

The concentrated product was applied at 75°C onto a chromatographic equipment (ISMB) with ion exchange resin Dianion UBK 550 in Sodium form, for obtaining a fraction enriched in maltose. Said product had the following composition (DP1: 1.1%; DP2: 96%; DP3: 1.7%; DP4+: 1.2%).

Further details are displayed in Table 2

**Table 2**

| Results expressed per hour and per m³ of resin | | | | | |
|---|---|---|---|---|---|
| | | Feed | Product enriched in maltose | Co-product | Water |
| Composition (%) | DP1 | 1.5 | 1.1 | 2.4 | |
| | DP2 | 80.0 | 96.0 | 41.1 | |
| | DP3 | 12.5 | 1.7 | 38.7 | |
| | DP4+ | 6.0 | 1.2 | 17.8 | |
| Total weight (kg/h) | | 37.6 | 41.48 | 71.65 | 75.36 |
| Flow rate (L/h) | | 29.00 | 35.30 | 69.1 | 75.36 |
| % d.s. | | 60.0 | 38.5 | 9.2 | |

| | | | | | |
|---|---|---|---|---|---|
| The yield of the product enriched in maltose is (total weight * % d.s. product *100/ total weight * % d.s of feed) = 70.8%. | | | | | |

### Example 5

### Hydrogenation

21.6 Kg (52% dry substance) of the fraction enriched in maltose having a composition (DP1: < 1.0%; DP2: 96-98%; DP3: <2%; DP4<1) was charged into a stainless steel hydrogenation reactor. Activated nickel catalyst was added in an amount of 3.6% on dry matter of the fraction enriched in maltose and the suspension was vigorously stirred and heated up to 135°C under hydrogen pressure of 43 bar. After 180 minutes of hydrogenation, the suspension was cooled to 90°C and the catalyst was removed by settling and filtration. The aqueous solution at temperature of 40°C was ion exchanged and polished over cationic and anionic resins and granular carbon.

The product obtained had the following composition (HPLC analysis: Bio-Rad Aminex HPX-87, cation exchange column is the calcium form, column temperature: 80°C, Eluent Flow Rate: 0.6 ml/minute, column pressure limit: 1200 psi, injection volume: 20 µL, pressure control limit about 200 psi above the normal operating pressure of the column, eluent: degassed Milli-Q Purified water, detector: Differential refractometer)

| | |
|---|---|
| Hydrogenated DP1 (sorbitol): | 1.1% |
| Hydrogenated DP2 (maltitol) : | 95.8% |
| Hydrogenated DP3: | 1.5% |
| Hydrogenated DP4+: | 1.2% |
| Others: | 0.4% |

### Comparative example 5 - Hydrogenation - see EP 1 656 388

21.6 Kg (52% dry substance) of the fraction enriched in maltose having a composition (DP1: 1.1%; DP: 96%; DP3: 1.7%; DP4+: 1.2%) was charged into a stainless steel hydrogenation reactor. Activated nickel catalyst was added in an amount of 4% on dry matter of the fraction enriched in maltose and the suspension was vigorously stirred and heated up to 135°C under hydrogen pressure of 43 bar. After 180 minutes of hydrogenation, the suspension was cooled to 90°C and the catalyst was removed by settling and filtration. The aqueous solution at temperature of 40°C was ion exchanged and polished over cationic and anionic resins and granular carbon. The product obtained had the following composition (HPLC analysis: Bio-Rad Aminex HPX-87, cation exchange column is the calcium form, column temperature: 80°C, Eluent Flow Rate: 0.6 ml/minute, column pressure limit: 1200 psi, injection volume: 20 µL, pressure control limit about 200 psi above the normal operating pressure of the column, eluent: degassed Milli-Q Purified water, detector: Differential refractometer)

| | |
|---|---|
| Hydrogenated DP1: | 2.1% |
| Hydrogenated DP2: | 94.8% |
| Hydrogenated DP3: | 1.5% |
| Hydrogenated DP4+: | 1.2% |
| Others: | 0.4% |

## Claims

1. A process for preparing a maltitol containing syrup comprising the successive steps of
a) Carrying out liquefaction of a starch milk,
b) Carrying out saccharification of the liquefied starch milk in the presence of alpha-amylase, and beta-amylase and a debranching enzyme selected from the group of pullulanase, iso-amylase and mixtures thereof,
c) Further adding maltogenic alpha-amylase and/or iso-amylase, optionally followed by demineralisation of maltose containing syrup,
d) Molecular sieving of the maltose containing syrup to obtain a fraction (A) comprising at least 95% maltose based on dry substance of fraction (A), and
e) Hydrogenating catalytically fraction (A) for obtaining a liquid maltitol enriched product (B);
wherein in step b) the saccharification is taking place in presence of from 1% to 4% of residual activity of total amount of alpha-amylase applied in the liquefaction of step a);
and wherein after step c) additional alpha-amylase is added at about 70 to 85% spent time of total saccharification time, for obtaining a maltose containing syrup comprising at least 85% maltose based on dry matter and less than 1.5% glucose based on dry matter.

2. The process according to claim 1 wherein the molecular sieving of step d) is a chromatographic fractionation.

3. The process according to any one of claims 1 or 2 wherein in step b) the ratio of beta-amylase to debranching enzyme is from 1:1 to 1:4.

4. The process according to any one of claims 1 to 3 wherein in step b) the debranching enzyme is pullulanase.

5. The process according to any one of claims 1 to 4 wherein in step a) the liquefaction is taking place until a DE of not more than 6.

6. The process according to any one of claims 1 to 5 wherein in step c) the addition of maltogenic alpha-amylase and/or iso-amylase is taking place at about 20 to 50% spent time of the total saccharification time.

## Patentansprüche

1. Verfahren zum Zubereiten eines Maltitol-haltigen Sirups, umfassend die aufeinanderfolgenden Schritte von
a) Ausführen einer Verflüssigung einer Stärkemilch
b) Ausführen einer Verzuckerung der verflüssigten Stärkemilch in Gegenwart von Alpha-Amylase und Beta-Amylase und eines Verzweigung abbauenden Enzyms, ausgewählt aus der Gruppe von Pullulanase, Iso-Amylase und Gemischen davon;
c) weiterem Zugeben von maltogener Alpha-Amylase und/oder Iso-Amylase, optional gefolgt von Demineralisierung von Maltose-haltigem Sirup,
d) Molekularsieben des Maltose-haltigen Sirups, um eine Fraktion (A) zu erhalten, die mindestens 95% Maltose umfasst, bezogen auf eine Trockensubstanz von Fraktion (A), und
e) katalytisches Hydrieren von Fraktion (A), um ein flüssiges Maltoseangereichertes Produkt (B) zu erhalten;
wobei in Schritt b) die Verzuckerung in Gegenwart von 1 % bis 4 % Restaktivität einer Gesamtmenge an Alpha-Amylase, die in der Verflüssigung von Schritt a) angewendet wird, stattfindet;
und wobei nach Schritt c) zusätzliche Alpha-Amylase bei etwa 70 bis 85 % abgelaufener Zeit der gesamten Verzuckerungszeit zugegeben wird, um einen Maltose-haltigen Sirup zu erhalten, der mindestens 85 % Maltose, bezogen auf die Trockensubstanz, und weniger als 1,5 % Glukose, bezogen auf die Trockensubstanz, umfasst.

2. Verfahren nach Anspruch 1, wobei das Molekularsieben von Schritt d) eine chromatografische Fraktionierung ist.

3. Verfahren nach einem der Ansprüche 1 oder 2, wobei in Schritt b) das Verhältnis von Beta-Amylase zum Verzweigung abbauenden Enzym von 1:1 bis 1:4 ist.

4. Verfahren nach einem der Ansprüche 1 bis 3, wobei in Schritt b) das Verzweigung abbauende Enzym Pullulanase ist.

5. Verfahren nach einem der Ansprüche 1 bis 4, wobei in Schritt a) die Verflüssigung bis zu DE von nicht mehr als 6 stattfindet.

6. Verfahren nach einem der Ansprüche 1 bis 5, wobei in Schritt c) die Zugabe von maltogener Alpha-Amylase und/oder Iso-Amylase bei etwa 20 bis 50 % abgelaufener Zeit der gesamten Verzuckerungszeit erfolgt.

## Revendications

1. Procédé de préparation d'un sirop contenant du maltitol comprenant les étapes successives consistant à
a) effectuer une liquéfaction d'un lait d'amidon.
b) effectuer une saccharification du lait d'amidon liquéfié en présence d'alpha-amylase, de bêta-amylase et d'une enzyme de déramification choisie parmi le groupe constitué de pullulanase, iso-amylase et leurs mélanges,
c) ajouter de plus une alpha-amylase et/ou iso-amylase maltogéniques, facultativement suivi d'une déminéralisation du sirop contenant du maltose,
d) effectuer un tamisage moléculaire du sirop contenant du maltose pour obtenir une fraction (A) comprenant au moins 95 % de maltose sur la base de la substance sèche de la fraction (A), et
e) hydrogéner de manière catalytique la fraction (A) pour obtenir un produit liquide enrichi en maltitol (B) ;
dans lequel, à l'étape b), la saccharification a lieu en présence de 1 % à 4 % d'activité résiduelle d'une quantité totale d'alpha-amylase appliquée lors de l'étape de liquéfaction a) ; et
dans lequel, après l'étape c), une alpha-amylase supplémentaire est ajoutée entre environ 70 et 85 % de temps écoulé du temps total de saccharification, pour obtenir un sirop contenant du maltose comprenant au moins 85 % de maltose sur la base de la matière sèche et moins de 1,5 % de glucose sur la base de la matière sèche.

2. Procédé selon la revendication 1, dans lequel le tamisage moléculaire de l'étape d) est un fractionnement chromatographique.

3. Procédé selon l'une quelconque des revendications 1 ou 2, dans lequel, à l'étape b), le rapport entre la bêta-amylase et l'enzyme de déramification est compris entre 1:1 et 1:4.

4. Procédé selon l'une quelconque des revendications 1 à 3, dans lequel, à l'étape b), l'enzyme de déramification est une pullulanase.

5. Procédé selon l'une quelconque des revendications 1 à 4, dans lequel, à l'étape a), la liquéfaction a lieu jusqu'à un DE de pas plus de 6.

6. Procédé selon l'une quelconque des revendications 1 à 5, dans lequel, à l'étape c), l'ajout d'alpha-amylase et/ou iso-amylase maltogéniques a lieu entre environ 20 et 50 % de temps écoulé du temps de saccharification total.
